# EUROPEAN PATENT APPLICATION

(11) **EP 2 016 918 A1**
(43) Date of publication of application: **21.01.2009**
(21) Application number: 07380327.2
(22) Date of filing: 23.11.2007
(51) Int. Cl.: A61B 17/80

(54) **Blocked plate with combined holes, stability control and double angulation, to join fractured bones**

(30) Priority: 18.07.2007 AR P070103195
(71) Applicant: Pizzicara, Mario Angel, Luis Graneo 2955 B° Primera Junta 5000-Ciudad de Cordoba (AR)
(72) Inventor: Pizzicara, Mario Angel, Luis Graneo 2955 B° Primera Junta 5000-Ciudad de Cordoba (AR)
(74) Representative: Polo Flores, Luis Miguel

(57) **Abstract**

It provides a plate with a plurality of holes formed based on the combination of two identical cylindrical holes (2,3).

Said cylindrical holes are provided with conical threads (b) and besides, one of them is provided with a descending spherical milling (4) that make up a grading (p) that starts over the initial center (a1) with an initial depth (f1) and finishes over the ending center (a1.1) with a final depth (f2).

A corresponding quantity of compressive screws with rounded head (5) enable the adjustment from an initial position on the initial center (a1) up to a final position in the end center (a1.1) and accepts their replacement for blocked screws with conical thread (6) located in said final position.

## Description

This invention consists of a **Blocked Plate with combined holes, stability control and double angulation, to join fractured bones.-** With the purpose to make this invention understandable, so that the same can be carried into practice easily, a precise definition will be given in the paragraphs below with the preferred manner of achievement making reference in the same to the illustrative drawings that are hereby enclosed. All of this as a simply demonstrative example but not limiting to the invention, which components can be selected from several equivalents without drawing away from the principles of the invention stated in this documentation.
PRIOR ART: The inventor knows several achievements which are below analyzed.-As a consequence of this, Patent number: BG64612 has been found, which consists in an Internal Accessory for Osteosynthesis. The accessory is applied in traumatology for long tubular bones in the parts next to the diaphysis and distal area, as well as in fractures of short tubular bones in case of spiral type fracture, particularly in fractures made fragments without intermediate fragment. It contributes to obtain the stable fixing of the bones and the fragments because of its complete diametrical grip in the area and around the fracture by means of the additional screws that fix the bones and the bones fragments, and the screws for the stable location of the accessories. The piece that has a bigger area lets the fragments to be located in it and to be fixed by the screws.The accessory comprises two semi-cylindrical plates to adjust the bone, and both pieces of the plate ends are adjusted together by means of clamps.

The semi-cylindrical plates comprise two portions (1 and 2), different from the surface, but they have the same length and curvature, part (2) is manufactured with the teeth (3) which are self aligned in the respective windows (4) machined in part (1) in a mounted manner, and the other ends of the two pieces are interconnected by means of the holes (5) and the screws (6). The longitudinal section (8) and the openings (9) for the screws that fix are machined on the surface of both pieces (1 and 2). Patent Number CA 1160927 is also studied, which comprises a compatible inner Plate, which is a fixing inner plate in order to build a bridge over two sections of a fractured bone which is formed to have the characteristics in terms of flexion, axial and torsion creating in this way a convenient situation for the bone which is repaired. These characteristics include high resistance to flexion and to torsional stress, and relatively low axial rigidity. One plate configuration that has been determined to have the desired characteristics of rigidity is a metal plate or an elongated flat hollow tube. The adjustments are provided in such a manner as to avoid movement of the bone, and this can be obtained filling the member of a medically inert substance, such as silicon rubber or ultra-high molecular weight polyethylene, or sealing the openings in the tubular plate. The material of the bone fixing plate can be selected according to their mechanical characteristics and also as biologically inert substances that do not have adverse physiological effect when they are grafted in the human body.
Patent Number CN1647775 is also studied, which consists of an absorbable bone plate, metal bone plate for bone fixing operation under double wing cross locking torque, the device for the bone fixing operation includes the absorbable plate manufactured with an absorbable material and a metal plate to improve the periosteum source of blood and the corn formation, and is used to internally fix the fracture ends. The absorbable plate and the metal plate can be joined together or they can be joined to the available bone plate to get the coupler stable and steady to the torsion stress with two fixing wings without the need of a secondary operation. When the lateral wing type or the absorbable plate type are combined or joined with the Ti-Ni memory alloy, the secondary operation can be omitted. The absorbable plate and the metal plate with the raised notch structure of the beam can ensure the mechanical force, provide the conditions to repair the periosteum blood source at the end of the fracture and promote the callus formation. Besides, this invention has reduced costs.
Patent Number DE102005034068 is also studied, which consists of a partially absorbable Osteosynthesis device for example for the ostesynthesis, that has an osteosynthesis plate connected to the bone on both sides of the fracture and the plate is made of an absorbable material and is reinforced with the help of stretching elements. The device has an ostesynthesis plate connected with a bone on both sides of the fracture. The ostesynthesis plate is made of an absorbable material and is reinforced with the help of tensing elements. The plate is connected reciprocally and the ostesynthesis has an absorbable material reinforced by a help. The screws holes are provided (11) in the absorbable material and the plate is made of metal. The help is designed since the spatial or laminate net is tighten shaped. The help is made of metal material such as chromium, nickel, titanium or plastic.

Patent Number DE19821680 is also studied, which consists of a Plate for mounted bones in order to support the fractured bones together to heal, the mounting for a bone plate is generally a wire U-shaped clip with two parallel bolts. The limb that is being joined, between the bolts (6,7) has a curvature (4.5.8) that is bent far from the line that connects (VL) between the bolts. At least one of the bolts (6.7) has a limit stop, and the bolts have arranged the limbs (2.10). The clip (1) is made of wire, that can be magnetized.
Patent Number DE3538238 is also studied, which consists of a ostesynthesis fixing Element. This invention is related with an element for the ostesynthesis, specially for the fixing of bone fragments by means of a supporting plate. The fixing element comprises a pin sleeve and a screw that holds to the pin sleeve, the pin sleeve is made of a viscoplastic biocompatible material and the outer surface has a thread. The pin sleeve can be slightly separated from the end that faces the head of the screw and can be separated more in its other end, and creates a simple and steady assembly in the bone tissue. Together with a metallic screwdriver the pin sleeve can be supervised constantly during the assembly with respect to the screen of the x-ray or jointly with a scale that measures.
Patent Number EP0013862 is also studied, which consists of an Osteosynthesis Plate. The plate for osteosynthesis consists of a reinforced carbon fiber synthetic material, the carbon fibers (12) are distributed in layers.On the lower side (13) to face the bone there is a layer (14) of about 1 millimeter thick, that has few or none carbon fibers and consequently it has an elasticity module comparable to the bone or reduces the increase of the carbon fiber from layer to layer so that the top layer (15) has an elasticity module comparable to that of the metal conventional plates. In order to make up the plate easily by means of hot air current, a thermoplastic synthetic material is used.
Patent Number EP0206767 is also studied, which consists of fixing bone Plates. The healing of a fracture is beneficially influence using fixing bone plate (10) formed by stainless steel with the holes (11) for the screws that fix and covered with the material electrically of flexible insulation (13) at least over a face (14) provided to be placed over the bone, the preferable layer and conveniently exceeding that also extends on all the other surfaces (16, 17,18) of the plate, with the exception of those surfaces inside the screw holes (11) that will be in contact with the fixing screws, that the surfaces go on being with naked metal. Patent Number ES2027846 is also studied, which consists in Improvements in the object of the main patent 9001854, for: "vertebral osteosynthesis plate", this consists of a cuboid elongated body made up of two equivalent pieces, connected by means of a junction with an inserted bar in the corresponding holes, made in the respective ends of these pieces, and there are radial grooves in the covering and juxtaposed areas which determine fixed and invariable positions of the articulated pieces, and that determine the angles that are also fixed and the distance between the openings of the free ends of said articulated pieces.

Patent Number FR2692140 is also studied, which consists of a partial Prostheses to replace the knee joint - it comprises the condylar component implanted in the lowe4 end of the femur cooperating with the tibial plate supported in the inserted-bone bar. The prosthesis includes a femoral condylar component (5) that cooperates with a tibial plate (6). A metal bar member (4) is implanted transversally in the higher part of the tibia, with a number of points of projection downwards (7) to anchor in the bone spongy tissue 9).
The tibial prosthesis plate is against the upper surface of the bar and it is made up with a slightly concave upper surface (9) to cooperate with the condylar component. The condylar component, which is implanted on the thin tissue of the femur lower end, has a lower concave surface for the rotational sliding with the tibial plate. ADVANTAGE - improved anchorage of the bone and carried characteristics.
Patent Number FR2706763 is also studied, which consists of an Ostheosynthesis Plate, which is made up with a metal alloy which has an elasticity module near to the bone, it has a 3,5 and 4,5 millimeter thickness and includes the ends beveled (6).
Patent Number FR2709410 is also studied, which consists of an Osteosynthesis clamp Plate Number, the clamp plate (10) consists of a curved plate (11) equipped of the points (12), oblong holes (13) for the pitch of the lock that screws (2), and a threaded hole (14) to lead the impact with the help of an impactor/positioner that its base levels the curved shape of the plate (11). Main Use: surgery.
Patent Number FR2726461 is also studied, which consists in a ostheosynthesis compressive Plate for long boles. The plate has holes (1 a) to receive the screws that they fix (2) jointly with the washers (3) to fix the plate in any section of the fracture, so that the body weight crosses the fracture area. The holes and washers are of a similar dimension to compose a fixed assembly. Alternatively, they can be different to let the washers move on the plate.
Clamp plate to bones for osteosynthesis that have a trim around the screw thread for and adjustment that is opened inside the Plate Number of Patent:

FR2790198 The plate clamp screw for the bone osteosynthesis has 5) trims of the sleeve (around the screw thread). The adjustable sleeve can be mounted on a plate hole (3). The sleeve extends radially under the effect of compression of the screw shaft during the screwing to allow the shaft to be fixed in an angle.

Patent Number FR2835733 is also studied, which consists in Osteosynthesis fixing for the vertebras that have a flat plate with a hole to receive the threaded shaft of the screw that fixes and grooves to receive the ends of jaws. Osteosynthesis fixing for vertebras that have a flat plate (3) with a hole (4) to receive an threaded shaft to fix screw (5). The plate has a rectangular groove (20) secant to the hole axis, and at least two slots (31.32) located on both faces opposed (33.34) to the plate. Both slots are formed to receive the ends of (35.36) two jaws (37.38) with a fixing traction of the reduction.
Patent Number FR2860139 is also studied, which consists of a whole plate device for bones for orthopedic surgery, which has a screw through hole and clamp inside the bone, and this has a screw thread with an propeller angle less than six grades to give irreversibility statically to the screw. The device has a screw (2) a through hole (3) with a bone plate (1) and this anchors to the bone, to make up the plate in the bone. The hole has a milling (4) to receive a cylindrical part (8) of a screw head (5). A screw thread (6) that has an propeller angle less than six grades to give statically irreversibility to the screw thread.
The Improvements to the metal plates for fractured bones that correspond to Patent Number GB191128864 have also been studied: where a metal bar 1 to join the fractured bone parts is made with a longitudinal groove 2 and slots 3 that hook with the sawn washers 4 with the screws 7. The washers are formed with the central ribs disposed that lock to slot 2. In a modification sown in Figure 7, the sides of slot 2 are notched to hook slots 6' in the ribs 5' of the washer.

Patent Number IT1267520 is also studied, which consists in compressive plate to restore the cohesion between the tibial plate and the lower part of the bone. This invention is related with a compressive plate that can favor the cohesion restoration between the tibial plate and the bone after the surgery to locate said plate. The plate according to the invention comprises two metal bars that are connected in a stable manner together with the possibility that one can be moved with respect to the
other due to their prismatic device, in such a manner that an extensible force can be applied at the proper time to both end bones that have to be joined, which are accordingly fixed.
Patent Number JP8206143 is also studied, which consists of the bone plate and bone screw. This plate comprises a body 1 made of a biocompatible metal, such as, stainless steel, titanium or a titanium alloy and a beveled edge 2 around. The rear bone line 3 the plate is slightly curved to follow the shape of a spine. The front edge 4 is hollowed to the inside to be given a cut 5 in order to ease the drive of the bone plate with a device such as forceps and both edges are smoothly rounded. The traversing surface of the bone plate is curved to lead the surface of a bone and of the top surface shapes 6 of these plates or dual faces. The lower surface that gets in contact with the bone presents a slightly curved surface, preferably cylindrical.
Patent Number KR20030033118 is also studied, which consists in metal Plate with elastic part for bone fixing. Metal Plate with elastic section for bone fixing that is provided to prevent osteoporosis which is shown after fixing the metal plate due to the physical force of the fracture partition. COMPOSITION: Metal plate with an elastic section for bone fixing includes a structure that has elasticity between the screw holes. Consequently the force that will be concentrated on the fracture piece is generally dispersed, while the structure resists the lack of metal. The metal plate has also a sufficient initial fixing and the metal fracture does not happen-

Patent Number MXGT03000010 is also studied, which consists of a Plate for Osteosynthesis. A metallic implant made of steel or titanium for medical use and applied to bones, preferably to the portions next to the tibia and the humerus in fractures and short bones (correcting osteotomies) which require a strong fixing to recover immediately the limb movement, obtaining in such a manner the bone consolidation or a callus formation for the limb rehabilitation, which enables the person to get back to his daily activities faster. The bone implant is characterized in that it comprises a plate 55 mm long, that has 10 mm diameter and 4 mm thickness, said plate includes three longitudinal openings 10 mm long and 7 mm diameters, a small groove located between the first and the second opening, which represents the implant placing with respect to the fracture track or to the bone cut location. The plate includes a slightly angular shape on the lateral surface similar to an italic S, said angulation is presented only in the third half of the plate that has a dislocation according to the 10 mm to cover, in such a manner the remaining next right and distal of the third of this. A useful screw to fix the plate to the bone is located in each opening, the first screw is driven upwards and downwards and to the rear position, the second screw is driven downward and upward and to the forward portion, so both screws provide the compression on the extreme upper lateral of said plate, thus improving the fracture stability. The third screw is driven perpendicular to the plate to provide the Inter.-fragment dynamic compression. The chosen plate includes a sheet the nearest located of this, the plate and the sheet make up a 90 degree angle, the last conclusion of the element in a selection and having a about 30 to 40 mm length, the above mentioned depending on the place of use as well as the bone depth; said sheet has below 3 mm thickness and ends in a 1 mm section, which provides an initial lock for the implant and contributes to the first location of this with respect to the bone.

Patent Number PT 103215 is also studied, Which consists in the system for Osteosynthesis fixation used in the treatment of fractures in oblong tubular bones that have a compression belt that can be placed, fixed with clamps and closed in the grooves made up in the othopedic plates to keep the system without movement. The system includes the orthopedic plates formed with the grooves and the small holes. A compression belt can be seated, fixed with clamps and closed in the grooves of the orthopedic plates to keep the system with no movement.
Patent Number UA 7829U is also studied, which consists in the Device for the osteosynthesis with the help of a metal plate. The device for the osteosynthesis with the help of a metal plate consists in the conductor to deliver the screws with the guide to carry the plate with the puncture of the soft fine tissues.

Patent number US6241731 is also studied: Which consists in the Assembly of plate and screw to fix the bones, this assembly includes a plate and at least a screw, the plate includes at least a hole which defines a cavity to contain the head of the screw and the head of the screw provides a stopper resistant to keep the head of the screw inside the cavity with the rotatory movement free but it is prevented from any axial movement, to avoid that the screw moves from the plate hole.
It was also studied the Argentinian application P000106884 that referes to an osteosynthesis plate formed with an upper side and a bottom side which presents a pluralityof holes located along the longitudinal axis and intended to receive osteosynthesis screws where at least one of the holes presents a combination between a circular hole -14- with -d-diameter and a symetry center -Sk- and a longitudinal hole -24- with a symetry center of -S1- symetry which presents a greater axis -a-extended in the longitudinal directio of the plate and a smaller axis -b- - erpendicular to the previous one.
The distance between both symetry centers -Sk- and -S1- results smaller that the result of d/2 + a/2.
Due to what was previously stated, it can be concluded that all the previous mentioned documentation does not represent an obstacle for this application.

AIM: This invention is about a **blocked plate of combined holes, stability control and double angulation to join fractured bones.** It refers to a way of stabilizing bone fractures by means of a metal plate inserted in a surgical operation under the skin and on the bone and fixed to it with screws. Such operation hereinafter called "osteosynthesis with blocked plate" will allow the surgeon to compress the fracture and leave the osteosynthesis assembly only with blocked screws without the need for combining it with compressing screws.
It is necessary to explain that up to now, there are different ways of fixing bone fractures in the field of the medical technology. Among the different models of internal fixation plates there are those that have self-compression holes in which the screw or the screws are of the compressive type and the purpose is the fracture stabilization from the mechanical approach of the bone fragments among themselves while the plate is moved closer to the bone.
This technique is also known as reduction of the fracture GAP. It allows that the different bone parts, divided as a consequence of the fracture, remain closer among themselves thus allowing or making easier the osteosynthesis and consequently the consolidation.
There are also plates with threaded holes that offer a greater structural resistance and have the following advantages:
a) As the screws are tightened, they never force the thread on the bone, favoring the osteosynthesis success in bones with osteoporosis or of bad quality.
b) By giving an angular stability to the screws, the resistance to the extract or "all pull-out" is considerably increased.
c) The plate can be placed separated from the bone, with blocked screws, obtaining a greater circulation of blood and reducing the amount of dead tissue due to the contact of said plate with the bone.
Due to the variety of cases which a medical doctor has to face, it is necessary a sufficient versatile and reliable product to ensure, not only an effective performance during the surgical operation but also the accurate application of the most appropriate selected technique and the overcoming of the typical difficulties.
It has been demonstrated that in a plate to join bones in a definite manner (for stabilization of a fracture), the conjunction of compressive screws with blocked screws increases the stress to the level in which the compressive screws are placed in the plate, obtaining a fatigue increase in this area and a possible break of the plate at this level.
Another problem with the implants known up today is that if they are fixated with blocked screws ( the system that provides greater stability at present) the fracture GAP cannot be reduced with the plate and the screws in direct way.
As it has been explained before, the aim of this invention is to bring closer to the surgeon and the patient a plate to join bones in which the fracture GAP can be compressed with compressive screws that can then be replaced by blocked screws threaded to the plate thus to get an osteosynthesis assembly with uniformity of blocked screws and with the fracture GAP closed.

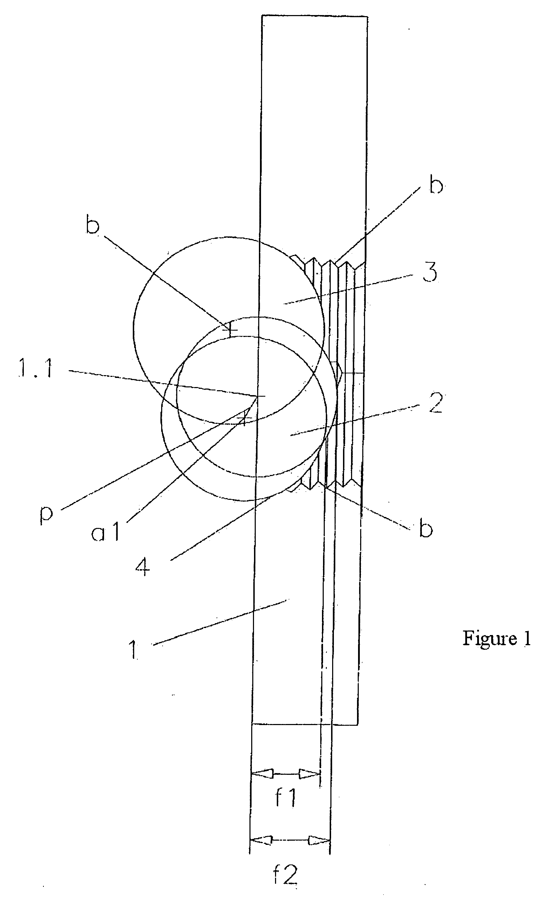

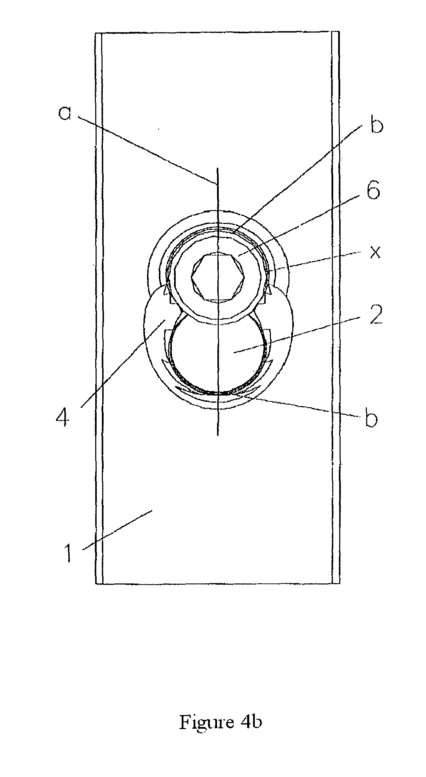

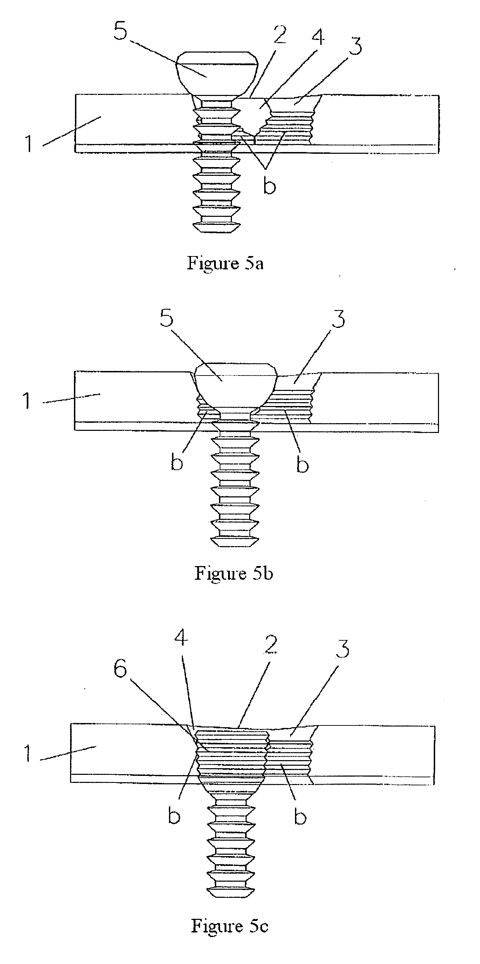

**DRAWINGS:** In the drawings attached to the pictures, the figure 1 outlines a cross cut in which the structure that makes up the hole combination is shown.

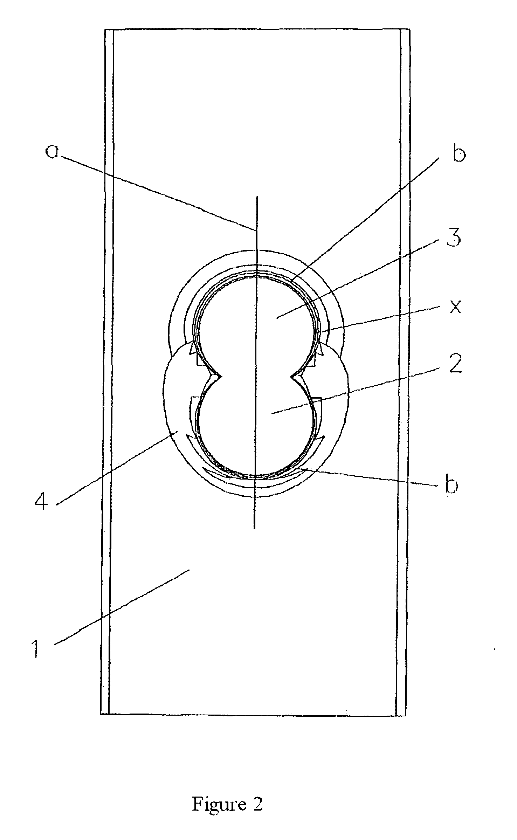

The figure 2 outlines a view in upper plant in which the hole distribution is shown.

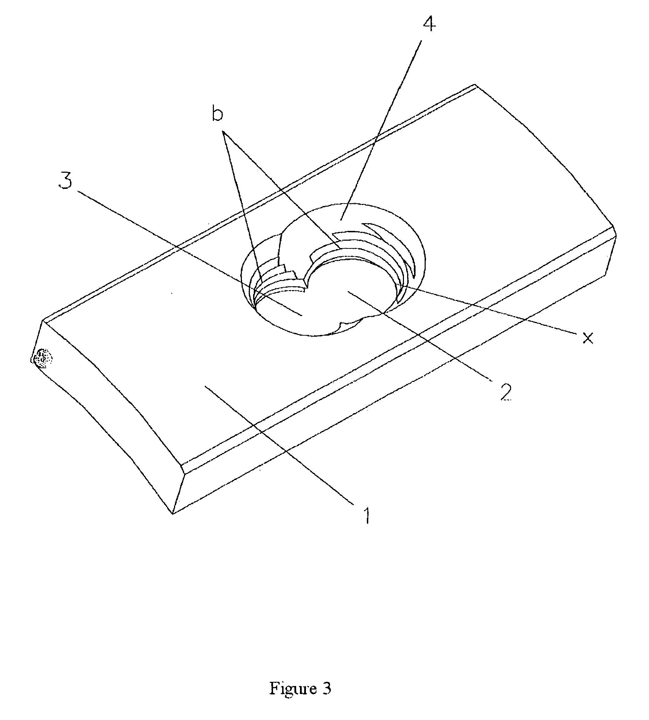

The figure 3 outlines a view in perspective of the plate with the combined hole.

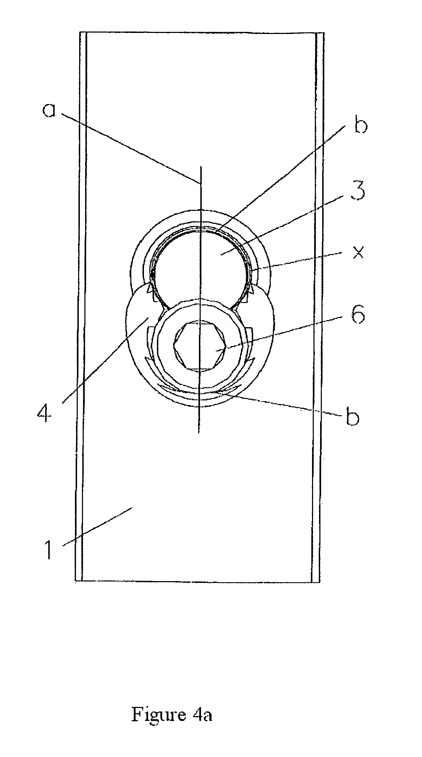

The figure 4a outlines a blocked screw of conical head thread in one of the two possible locations.
On the other hand the figure 4b outlines the same blocked screw of conical head thread in the remaining possible location.
Finally the figures 5a, 5b and 5c outline a sequence in which a plate is observed. The first two views / figures show different grades of compression achieved with a compression screw. The last figure allows to observe the plate in which a compression screw has been replaced by a blocked one with thread conical head.

**REFERENCES:** In the figures previously described the same reference character show the corresponding equal parts, being the number -1- the blocked plate; the number -2- a cylindrical hole ; the number -3- a second cylindrical hole, the number -4- a down spherical milling, the number -5- a compression screw with rounded head and the number -6-a blocked screw of conical thread.
The reference -a- has been reserved to indicate an axis of symmetry; the -b- for the thread conical ones; the -a1- for the start center; the -a1.1- for the end center, the reference -f1- for the start depth; the -f2- for the final depth, the reference -p- for an axis of the slope, and the reference -x- for the diameter in the lesser section of the cone.

**DESCRIPTION:** This development consists basically of a blocked plate with combined holes, stability control and double angulation, to join fractured bones, and, as can be seen is made up with the combination of two cylindrical holes, both of the threaded and one of them with descending milling.
Both holes have conical thread and their diameter in the smaller section of the cone is enough to permit without any inconvenient the pitch of the thread worm of the round headed and the thread headed compressive screws. The diameter of the later is bigger than the first ones.
A spherical milling in one of the holes gives to the plate the quality of "compressive".
The above mentioned quality allows the bone fractured sections as well as the bone fragments to get nearer. The rounded head compressive screw is placed in the initial center over a hole perpendicular to the plate and with pressure it goes down and fits over the end center position, and in this way it remains at level with the plate surface. Once the bone fragments are compressed (compression done by means of the rounded head screws), these can be taken out and exchanged by blocked screws with conical thread placed on the same point, being these screws more efficient in the fixing task for bone fractures. This is the usefulness of this combination of thread and housing for rounded head screws or "compressive".
OPERATION: Once the different components of the version of this invention are established, developed to explain their nature, the description is below complemented with the functional and operational relationship of its parts and of the result they provide.
With the aim to obtain a **Blocked Plate with combined holes, stability control and double angulation, to join fractured bones** over a blocked plate -1- of appropriate length, a plurality of holes have been practiced as can be observed in the schematic figures to adapt it to the bone structure to which it is intended according to the requirements of the fracture to be stabilized.

Each of this plurality of holes comes actually from the combination of two cylindrical holes -2- and -3-, both threaded, and one of them with a descending spherical milling-4-. This combination is basically an intersection of two identical holes (2 and 3) with an axis of symmetry -a- defined by an imaginary line that joins their centers. Both holes have a conical thread -b- which diameter -x- in the smaller section of the cone, turns out to be enough to permit without any inconvenient the pitch of the thread worm of both the compressive screws with rounded head -5- as well as the blocked screws with conical thread -6.The diameter of the later is bigger than the first ones. A spherical milling -4- with a gradient -p-, is started at the start center - a1- with an initial depth -f1- and finishes on the end center -a1-1- with a final depth -f2-. Confers to the blocked plate (1) the compressive quality. The above mentioned quality allows the bone fractured sections as well as the fragments (not represented), to get nearer. The compressive screw with rounded head (5) is fitted at an initial point on the start point (a1), that is to say, perpendicular to the plate, starting its adjustment to obtain its downward movement.-

With said pressure said compressive screw with rounded head (5) goes downward to finally stop over in the position of end center (a1.1), and finally remains nearly completely at the level of the surface of the blocked plate (1).
Once the bone fragments are compressed using for this the pressure exerted by the compressive screws with rounded head (5), these can be exchanged by blocked screws with conical thread (6) placing them in the same end center point (a1.1). In this way the fracture GAP can be compressed with rounded head compressing screws (5) so that later they are replaced by blocked screws with conical shape (6) that, screwed to the blocked plate (1), enable to obtain an osteosynthesis with uniformity of blocked screws with conical thread (6) that stabilize the position of the blocked plate (1) and separate it slightly from the bone keeping the fracture bone closed.

It is important to notice that dimensions as well as the position of the combined hole has variations.

Having described and determined the nature of the invention, its scope and the manner in which the same can be put into practice in its fundamental idea, the following is declared as invention and exclusive property:

## Claims

1. **Blocked Plate with combined holes, stability control and double angulation, to join fractured bones** of the kind that, through surgery, is placed under the skin and over the bone, of the kind that is fixed by means of screws that join it to the bone; **characterized** because said holes are made up based on the combination of two cylindrical holes (2,3) both of them provided with conical thread (b)and one of them with a spherical descending milling (4) with a gradient (p) that begins on the initial center (a1) with an initial depth (f1) and ends on the finishing center (a1.1) with a final depth (f2), where a corresponding quantity of rounded head compressive screws (5) are adjusted from an initial position in the initial center (a1) up to a final position in the end center (a1.1) and where its rounded head compressive screws (5) are replaced by blocked screws with conical thread (6) fixed in said final position.

2. Plate, according to claim number 1, **characterized** because the holes result from the intersection of two identical holes that have an axis of symmetry defined by means of an imaginary line that joins their centers.

3. Plate, according to claim number 1, **characterized** because the rounded head compressive screws (5) are replaced after compressing the bone fragments in the proper position in order to obtain the osteosynthesis.

4. Plate, according to claim number 1, **characterized** because the position of the rounded head compressive screw (5) in the initial center (a1) comes to be perpendicular to the blocked plate (1) and in the finishing center (a1.1) it remains nearly completely at level with the surface of said blocked plate (1).

5. Plate, according to claim 1, **characterized** because the diameter (x) in the smaller section of the conical thread (b) opens the pitch for the thread worm of both the rounded head compressive screws (5), as well as that of the blocked screws with conical thread (6).

6. Plate, according to claim 1, **characterized** because the blocked screws with conical thread (6) are screwed to the blocked plate (1) to obtain an osteosynthesis assembly stabilizing the blocked plate position (1) and separating it slightly from the bone keeping the fracture GAP closed.
